# EUROPEAN PATENT APPLICATION

(11) **EP 3 138 465 A1**
(43) Date of publication of application: **08.03.2017**
(21) Application number: 15847228.2
(22) Date of filing: 11.09.2015
(51) Int. Cl.: A61B 1/00, G02B 23/24

(54) **ENDOSCOPE BENDING TUBE AND ENDOSCOPE PROVIDED WITH ENDOSCOPE BENDING TUBE**

(30) Priority: 01.10.2014 JP 2014203508
(71) Applicant: OLYMPUS CORPORATION, Hachioji-shi Tokyo 192-8507 (JP)
(72) Inventor: NAKADE, Sho, Tokyo 192-8507 (JP); KURODA, Motohiro, Tokyo 192-8507 (JP)
(74) Representative: Schicker, Silvia
(86) International application number: PCT/JP2015/075883
(87) International publication number: WO 2016/052145

(57) **Abstract**

In an endoscope bending tube provided in a bending portion of an endoscope in which a first bending tube region 21a and a second bending tube region 21b having mutually different bending characteristics are arranged from a distal end side to a proximal end side of a bending portion 21, a slit width w1 of a plurality of bending slits 24 arranged in the bending tube region 21a is set to be smaller than a slit width w2 of a plurality of bending slits 25 arranged in the bending tube region 21b, a width of the bending slits on the distal end side between the neighboring bending slits is set to be smaller than a width of the slits on the proximal end side, and an interval between the neighboring bending slits of a slit row in the bending region located on the distal end side is set to be smaller than an interval between the neighboring bending slits of the slit row in the bending region located on the proximal end side.

## Description

### Technical Field

The present invention relates to an endoscope bending tube disposed in a bending portion that performs bending motion according to operation of an operation member provided in an operation section and an endoscope provided with the endoscope bending tube disposed in the bending portion.

### Background Art

Endoscopes inserted into subjects are widely used in medical and industrial fields in recent years.

Endoscopes used in the medical field in particular can be used to observe organs in a body cavity by inserting an elongated insertion portion into the body cavity which is the subject or perform, as required, various types of treatment using a treatment instrument inserted into an insertion channel for the treatment instrument provided in the endoscope.

A configuration of such an endoscope is known which is provided with a freely bendable bending portion in an insertion portion to improve insertability into a subject. A conventional bending portion generally incorporates a bending tube in which a plurality of bending pieces are rotatably connected and the bending portion is configured to be bent by remotely operating the plurality of bending pieces.

In addition to the aforementioned configuration, a bending portion in such a configuration is known which is provided with a bending tube which is a pipe-shaped metal tube or the like in which a plurality of slits are carved, the bending portion being bent by operating an operation section to cause an operation wire inserted in a wire guide provided in the bending tube to pull or slacken.

An endoscope inserted into, for example, the bronchus which has a multi-branched complicated structure is preferably configured such that the distal end region of the bending portion may be bent first to allow the insertion portion to be easily inserted into the back of the bronchus to make it easier to determine an insertion direction of the insertion portion.

Thus, for example, International Publication No. WO2013-19091 discloses an endoscope provided with a bending tube formed of a super-elastic tube body disposed inside the bending portion to allow the bending portion to be bent first.

That is, International Publication No. WO2013-19091 discloses a technique of an endoscope provided with a plurality of slits formed in a bending tube and a wire guide provided in an inner circumference of the bending tube, an interval between neighboring slits in a central region and a proximal end side region being set to be longer than an interval between slits in a distal end side region so as to allow the distal end side of the bending portion to be bent first.

However, the conventional endoscope disclosed in International Publication No. WO2013-19091 allows only the distal end side region of the bending portion to be bent first at a desired angle, whereas when the bending portion is further bent, the whole bending portion does not bend at the same radius of curvature.

For that reason, when the conventional endoscope is inserted into the body cavity such as the bronchus which has a multi-branched complicated structure, the proximal end side of the bending portion is bent so as to add a force to the bronchial wall, giving a patient an unnatural feeling, pain or the like, resulting in a problem that it is difficult to operate the insertion portion so as to turn in a small radius inside a subject.

Thus, the present invention has been implemented in view of the above-described circumstances and it is an object of the present invention to provide an endoscope bending tube configured to cause a distal end side to bend first before a proximal end side of a bending portion, cause, when the endoscope bending tube is bent in a maximum bending state, both the distal end side and the proximal end side of the bending portion to bend at the same radius of curvature to thereby allow an insertion portion to be easily inserted into a complicated subject, and an endoscope provided with the endoscope bending tube.

### Disclosure of Invention

### Means for Solving the Problem

An endoscope bending tube according to an aspect of the present invention is an endoscope bending tube having a longitudinal axis that extends from a distal end side to a proximal end side, the endoscope bending tube including a plurality of bending slits configured to penetrate from an outer circumferential surface to an inner circumferential surface side and extend in a circumferential direction, in which the plurality of bending slits are opened in rows at positions on one side and another side independently in a direction orthogonal to the longitudinal axis direction, and two or more bending tube regions along the longitudinal axis direction are formed of the slit rows made up of the plurality of bending slits opened at the positions on the one side and the other side, and a width of each of the bending slits of the slit rows of the bending region located on the distal end side is set to be smaller than a width of each of the bending slits of the slit rows of the bending region located on the proximal end side, and an interval between neighboring bending slits of the slit row of the bending region located on the distal end side is set to be smaller than an interval between neighboring bending slits of the slit rows of the bending region located on the proximal end side.

Furthermore, an endoscope according to an aspect of the present invention is provided with the endoscope bending tube in a bending portion.

### Brief Description of the Drawings

Fig. 1 is an overall view of an endoscope;
Fig. 2 is a perspective view illustrating main parts of a bending portion;
Fig. 3 is a cross-sectional view of main parts illustrating the bending portion along a longitudinal axis direction;
Fig. 4 is a diagram illustrating only a first bending tube region of the bending portion being bent upward;
Fig. 5 is a diagram illustrating the bending portion being bent upward up to a maximum bending state;
Fig. 6 is a diagram illustrating only the first bending tube region of the bending portion being bent downward;
Fig. 7 is a diagram illustrating the bending portion being bent downward up to a maximum bending state;
Fig. 8 relates to modification 1 and is a cross-sectional view of main parts illustrating the bending portion along the longitudinal axis direction;
Fig. 9 relates to modification 2 and is a cross-sectional view of main parts illustrating the bending portion along the longitudinal axis direction;
Fig. 10 relates to modification 3 and is a cross-sectional view of main parts illustrating the bending portion along the longitudinal axis direction;
Fig. 11 relates to modification 4 and is a cross-sectional view of main parts illustrating the bending portion along the longitudinal axis direction; and
Fig. 12 relates to modification 5 and is a cross-sectional view of main parts illustrating the bending portion along the longitudinal axis direction.

### Best Mode for Carrying Out the Invention

### (Configuration)

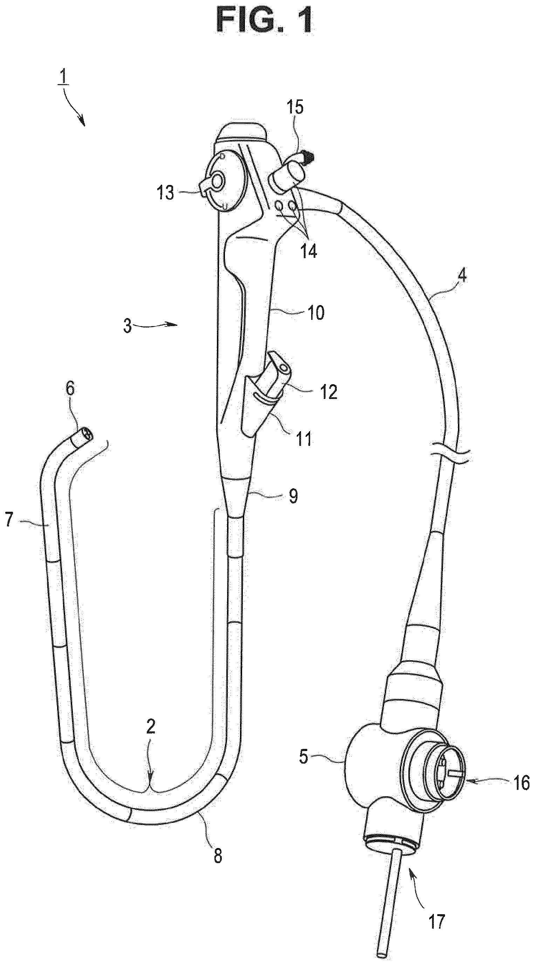

Hereinafter, an embodiment of the present invention will be described with reference to the accompanying drawings. As shown in Fig. 1, an electronic endoscope (hereinafter simply referred to as "endoscope") 1 according to the present embodiment is mainly constructed of an insertion portion 2 formed into an elongated tubular shape, an operation section 3 connected to a proximal end of the insertion portion 2, a universal cord 4 which is an endoscope cable configured to extend from the operation section 3, and an endoscope connector 5 disposed at a distal end of the universal cord 4 or the like.

The insertion portion 2 is a flexible tubular member formed by connecting a distal end portion 6, a bending portion 7 and a flexible tube portion 8 in order from the distal end side. Among the above-described parts, the distal end portion 6 accommodates an image pickup unit which is an image pickup apparatus which is not shown and incorporates image pickup means and an illumination section or the like.

The bending portion 7 is a mechanical region configured to be actively bendable in two directions; upward and downward (UP-DOWN) directions through turning operation of a bending lever 13 which will be described later among the operation members of the operation section 3.

Note that the bending portion 7 is not limited to the type but may also be of a type that can be bent in four directions including not only upward and downward directions but also leftward and rightward directions (all circumferential directions around the axis, through upward/downward, leftward/rightward operation, UP-DOWN/RIGHT-LEFT).

The flexible tube portion 8 is a tubular member formed with flexibility so as to be passively flexible. In addition to a treatment instrument insertion channel, various signal lines that extend from an image pickup apparatus incorporated in the distal end portion 6 and further extend from the operation section 3 to an interior of the universal cord 4, a light guide that guides illuminating light from a light source apparatus to be emitted from the distal end portion 6 or the like (all of which are not shown) are inserted through the flexible tube portion 8.

The operation section 3 is constructed of a bend preventing portion 9 provided on a distal end side and configured to cover a proximal end of the flexible tube portion 8 and be connected to the flexible tube portion 8, a grasping portion 10 connected to the bend preventing portion 9 and configured to be grasped by an operator by hand when using the endoscope 1, the operation section provided on an outer surface of the grasping portion 10 to operate various endoscope functions, a treatment instrument insertion portion 11 and a suction valve 15.

Examples of operation means provided in the operation section 3 include a bending lever 13 configured to perform bending operation of the bending portion 7, a plurality of operation members 14 configured to perform operations respectively corresponding to suction operation, image pickup means and illumination means or the like.

The treatment instrument insertion portion 11 is a component provided with a treatment instrument insertion port to insert various treatment instruments which are not shown and configured to communicate with a treatment instrument insertion channel inside the operation section 3 via a branch member. The treatment instrument insertion portion 11 is provided with a forceps plug 12 which is a cover member to open/close the treatment instrument insertion port and configured to be detachably (replaceably) attached to the treatment instrument insertion portion 11.

The universal cord 4 is a composite cable configured to insert various signal lines that pass through the interior of the insertion portion 2 from the distal end portion 6 of the insertion portion 2, reach the operation section 3 and further extend from the operation section 3 and to insert a light guide of the light source apparatus which is not shown.

The endoscope connector 5 is constructed of an electric connector section 16 provided on a side face portion to which a signal cable for connection with a video processor which is an external device which is not shown is connected, and a light source connector section 17 to which a light guide bundle which is not shown for connection with the light source apparatus which is an external device and an electric cable are connected.

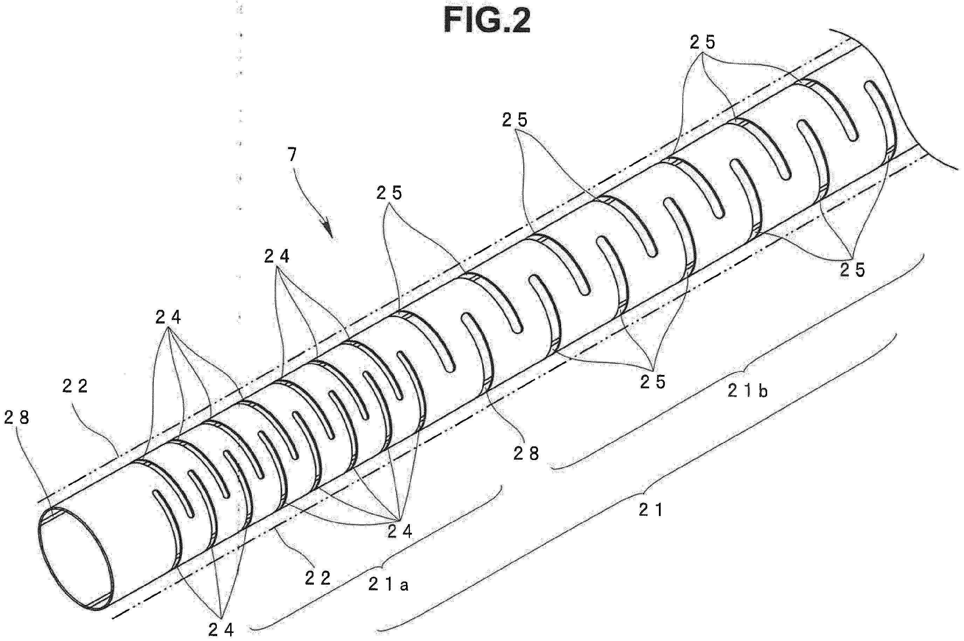

Here, a configuration of the bending portion 7 provided in the insertion portion 2 of the endoscope 1 according to the present embodiment will be described below based on Fig. 2 to Fig. 5. Note that description of a known configuration of the insertion portion 2, the distal end portion 6 and the flexible tube portion 8 will be omitted below.

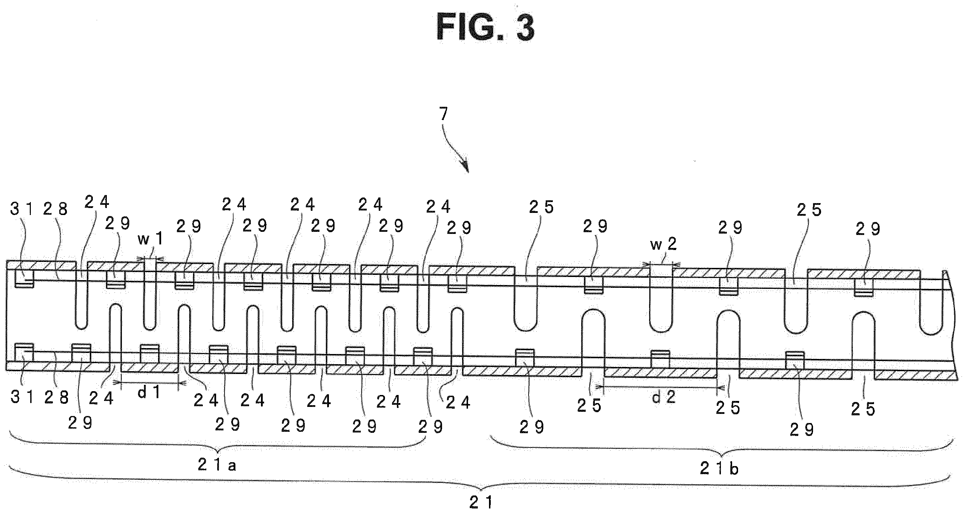

Fig. 2 is a perspective view illustrating a bending tube disposed in the bending portion and Fig. 3 is a cross-sectional view illustrating the bending tube. As shown in Fig. 2 and Fig. 3, the bending portion 7 is constructed of an outer cover 22 made of soft resin or the like and a metallic bending tube 21 covered with the outer cover 22. The bending tube 21 is configured using a cylindrical pipe member formed of a super-elastic alloy as a principal constituent. Examples of the super-elastic alloy member constituting the bending tube 21 include Ni-Ti (nickel titanium), titanium alloy, beta titanium, pure titanium, 64-titanium, A7075 and aluminum alloy.

A first bending tube region 21a and a second bending tube region 21b are set in the bending tube 21 in order from the distal end side as bending regions having different bending characteristics from each other.

A plurality of partially arc-shaped bending slits 24 penetrating from an outer circumferential surface side to an inner circumferential surface side of the bending tube 21 and extending in a circumferential direction of the bending tube 21 are provided in the first bending tube region 21a of the bending tube 21 at a predetermined interval along a longitudinal axis direction through, for example, laser machining.

Widths of the plurality of bending slits 24 in the longitudinal axis direction of the bending tube are set to w1, and in the present embodiment, the bending slits 24 are alternately formed at positions on one side and another side in a direction orthogonal to the longitudinal axis direction of the bending tube 21.

To be more specific, in the first bending tube region 21a, the plurality of bending slits 24 which are opened in the same direction are arranged in a row at an interval of d1 along the longitudinal axis direction of the bending tube 21 on the one side of the bending tube 21 corresponding to an upper side in the bending direction of the bending portion 7. On the other hand, in the first bending tube region 21a, the plurality of bending slits 24 which are opened in the same direction are arranged in a row at an interval of d1 along the longitudinal direction of the bending tube 21 on the other side of the bending tube 21 corresponding to a lower side in the bending direction of the bending portion 7. The respective bending slits 24 arranged on the one side of the bending tube 21 are arranged alternately with respect to the respective bending slits 24 arranged on the other side of the bending tube 21.

A plurality of partially arc-shaped bending slits 25 penetrating from an outer circumferential surface side to an inner circumferential surface side of the bending tube 21 and extending in a circumferential direction of the bending tube 21 are provided in the second bending tube region 21b of the bending tube 21 at a predetermined interval along the longitudinal axis direction.

Widths of the plurality of bending slits 25 in the longitudinal axis direction of the bending tube are set to w2, and in the present embodiment, the bending slits 25 are alternately formed at positions on one side and the other side in a direction orthogonal to the longitudinal direction of the bending tube 21.

To be more specific, in the second bending tube region 21b, the plurality of bending slits 25 which are opened in the same direction are arranged in a row at an interval of d2 along the longitudinal axis direction of the bending tube 21 on the one side of the bending tube 21 corresponding to the upper side in the bending direction of the bending portion 7. On the other hand, in the second bending tube region 21b, the plurality of bending slits 25 which are opened in the same direction are arranged in a row at an interval of d2 along the longitudinal axis direction of the bending tube 21 on the other side of the bending tube 21 corresponding to the lower side in the bending direction of the bending portion 7. The respective bending slits 25 arranged on the one side of the bending tube 21 are arranged alternately with respect to the respective bending slits 25 arranged on the other side of the bending tube 21.

Thus, the bending tube 21 is provided with the slit row of the bending slits 24 and 25 opened in the same direction and arranged in a row on the one side of the bending tube 21 and the slit row of the bending slits 24 and 25 opened in the same direction and arranged in a row on the other side of the bending tube 21.

Here, the width w1 of each of the bending slits 24 provided in the first bending tube region 21a is set to be smaller than the width w2 of each of the bending slits 25 provided in the second bending tube region 21b. Furthermore, on the same slit row, the interval d1 between the neighboring bending slits 24 in the first bending tube region 21a is set to be smaller than the interval d2 between the neighboring respective bending slits 25 in the second bending tube region 21b. Moreover, a ratio between the slit widths w1 and w2 is set to be equal to a ratio between the slit intervals d1 and d2.

That is, regarding the widths of the plurality of bending slits 24 and 25 arranged in the same slit row, the width w1 of the bending slit 24 located at the most distal end of the bending tube 21 is set to be smaller than the width w2 of the bending slit 25 located at the most proximal end, and between the neighboring bending slits, the width of the bending slit on the distal end side is set to be equal to or less than the width of the bending slit on the proximal end side.

Furthermore, the above-described respective slit widths w1 and w2 and respective slit intervals d1 and d2 are set so as to satisfy a relationship of d1:w1=d2:w2. In this way, the slit rows formed of the plurality of bending slits 24 and 25 opened in the same direction are set such that the ratio between the slit width and the slit interval of the neighboring respective slits is kept constant. In other words, the ratio between the sum of the slit widths per unit length and the sum of the slit intervals is set to be constant on the same slit row.

Note that in the example shown in Fig. 3, although a partial section spanning both the first bending tube region 21a and the second bending tube region 21b exceptionally does not satisfy the aforementioned relationship, in order to also satisfy the aforementioned relationship in the section in question, the interval between the neighboring bending slit 24 and the bending slit 25 may be an intermediate value between d1 and d2, that is, an interval of (d1+d2)÷2.

A plurality of wire guides 29 are disposed on an inner circumferential part of the bending tube 21. The plurality of wire guides 29 are provided between the plurality of bending slits 24 and 25 at the upper and lower positions (one side and the other side) of the bending tube 21. Furthermore, wire fixing portions 31 are disposed at upper and lower positions corresponding to the respective wire guides 29 at the most distal end portion of the bending tube 21.

Two operation wires 28 are inserted through the respective wire guides 29 arranged in the upper and lower parts of the bending tube 21 respectively, the operation wires 28 being pulled or slackened through operation of the bending lever 13 provided in the operation section 3 to move forward or backward. Distal ends of the operation wires 28 are fixed by the wire fixing portions 31 respectively.

Note that the operation wires 28 are inserted through a coil tube which is not shown inside the flexible tube portion 8 (see Fig. 1) connected to the proximal end of the bending portion 7 and the operation section 3. The coil tube is a protective member, a distal end of which is disposed so as to be located on a boundary portion between the bending portion 7 and the flexible tube portion 8, configured to protect the operation wires 28 themselves and various components inside the insertion portion 2 and the operation section 3.

### (Action)

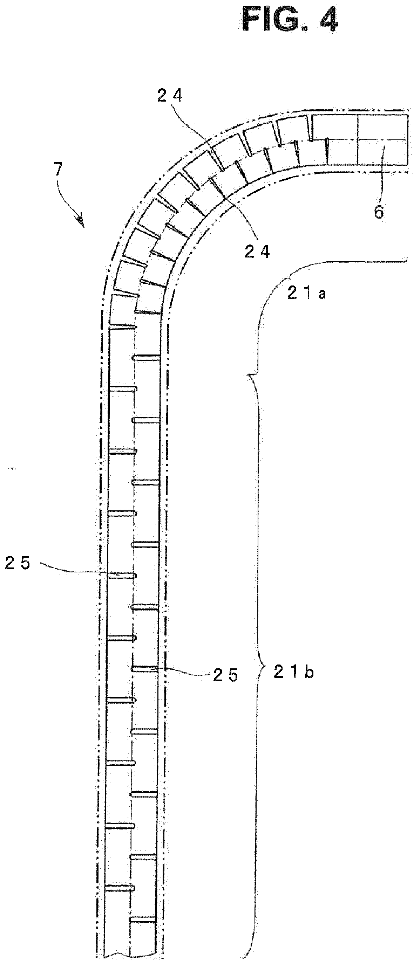

Action based on the aforementioned configuration will be described below using Fig. 4 to Fig. 7.

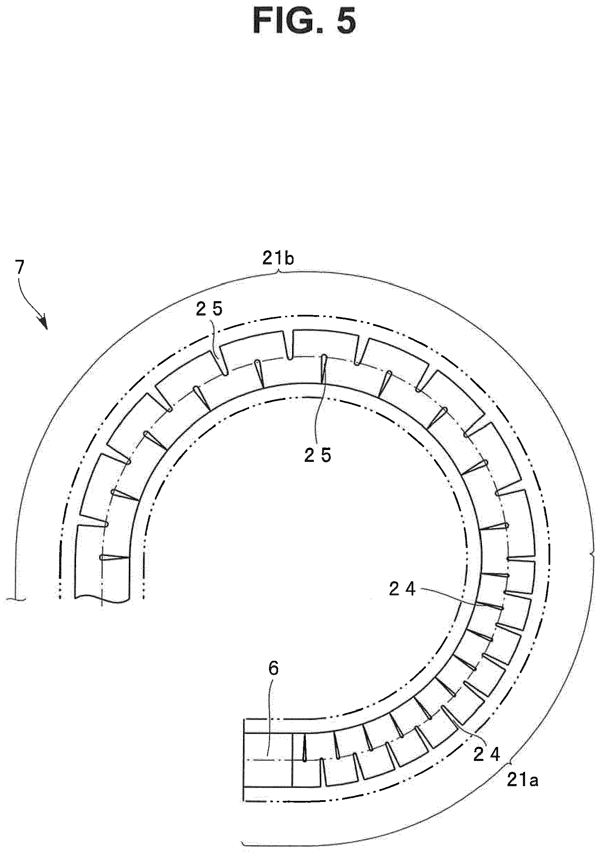

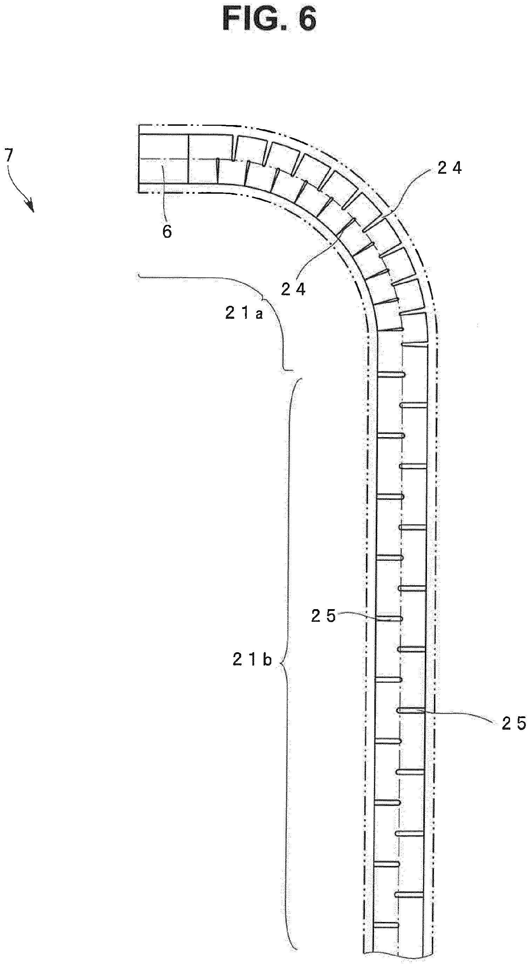

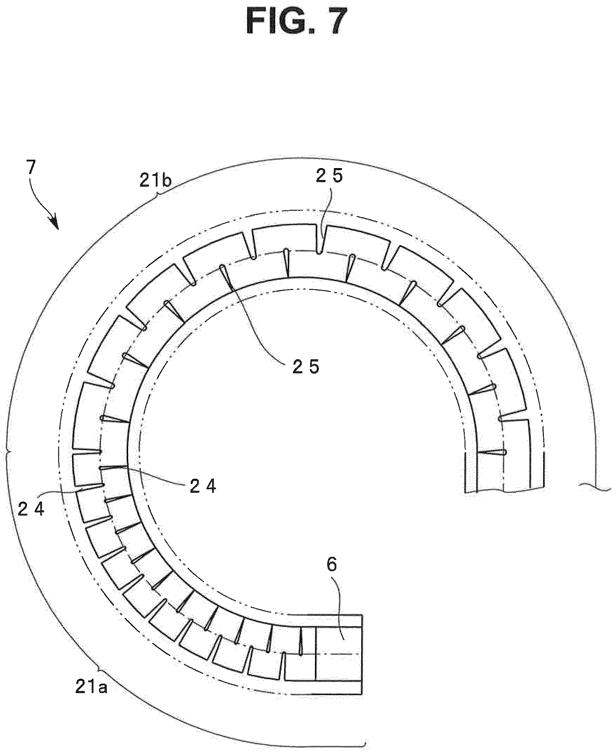

Fig. 4 illustrates the bending portion being bent toward an UP side, Fig. 5 illustrates the bending portion further being bent in the UP direction from the bent state in Fig. 4, Fig. 6 illustrates the bending portion being bent toward a DOWN side and Fig. 7 illustrates the bending portion in Fig. 6 being further bent in the DOWN direction.

When the bending lever 13 of the operation section 3 is turned in a predetermined direction and the two operation wires 28 on the upper and lower sides thereby move forward or backward, the bending portion 7 actively bends. That is, of the respective operation wires 28 fixed to the wire fixing portions 31 on the most distal end side of the bending tube 21 respectively, one is pulled and the other is slackened in conjunction with the turning operation of the bending lever 13 and the bending portion 7 actively bends as shown in Fig. 4 to Fig. 7.

As described above, the interval d1 between the neighboring respective bending slits 24 in the first bending tube region 21a in the same slit row of the bending tube 21 is set to be smaller than the interval d2 between the neighboring respective bending slits 25 in the second bending tube region 21b. That is, more slits per unit length are provided in the first bending region tube 21a than in the second bending tube region 21b, and the bending tube 21 is consequently set so that bending rigidity of the first bending region tube 21a is lower than bending rigidity of the second bending tube region 21b.

As a result, in an initial motion of the bending portion 7 when the bending lever 13 of the operation section 3 is operated, the first bending tube region 21a disposed on the distal end side of the bending tube 21 starts a bending motion first as shown in Fig. 4 or Fig. 6, and reaches a maximum bending state (maximum bending angle). When the bending lever 13 is further operated, the second bending tube region 21b disposed on the proximal end side of the bending tube 21 of the bending portion 7 starts a bending motion later than the first bending tube region 21a and reaches a maximum bending state (maximum bending angle).

Fig. 5 and Fig. 7 illustrate the bending portion 7 which has been bent up to a maximum bending angle combining the bending motion of the first bending tube region 21a and the bending motion of the second bending tube region 21b.

As described above, such a maximum bending state is achieved when the first bending tube region 21a bends first, the first bending tube region 21a performs a bending motion up to a maximum bending angle and the second bending tube region performs a bending motion up to a maximum bending angle.

Thus, when the whole bending portion 7 is bent to the maximum angle, since the ratio of w1 and w2 which are widths of the bending slits in the longitudinal direction of the bending tube is set to be equal to the ratio of d1 and d2 which are intervals between the bending slits, the whole bending portion is bent at a uniform radius of curvature.

That is, each curvature of the first and second bending tube regions 21a and 21b of the bending tube 21 depends on each of the slit widths w1 and w2 which is a distance until the anterior and posterior slit walls forming the respective bending slits 24 and 25 come into contact with each other. Since the ratio between the slit widths w1 and w2 is set to be equal to the ratio between the slit intervals d1 and d2 as described above in the present embodiment, it is possible to make the total slit width per unit length consistent between the first bending tube region 21a and the second bending tube region 21b on the same slit row. When the bending portion 7 is in the maximum bending state, the curvature of the first bending tube region 21a thereby is equal to the curvature of the second bending tube region 21b.

### (Effects)

As described above, when the endoscope 1 according to the present embodiment is bent through the bending lever 13 of the operation section 3, the first bending tube region 21a located on the distal end side of the bending portion 7 provided in the insertion portion 2 starts bending before the second bending tube region 21b on the proximal end side. Therefore, in a process of inserting the insertion portion 2 into a complicated tube path such as the bronchus, it is possible to cause only a distal end side of the bending portion 7 to bend in a desired insertion direction first. Thus, after causing only the distal end side of the bending portion 7 to bend in the desired insertion direction and causing the distal end side to advance through the bent tube path, a further bending operation is performed through the bending lever 13, and it is thereby possible to cause the whole bending portion 7 to bend along with the bending shape of the tube path.

That is, when the insertion portion 2 is inserted into a complicated body cavity such as the bronchus, the endoscope 1 is configured such that the first bending tube region 21a on the distal end side starts bending before the second bending tube region 21b on the proximal end side of the bending portion 7, and the insertion portion 2 can thereby turn in a small radius.

At this time, since the ratio between w1 and w2 which are widths of the bending slits in the longitudinal axis direction of the bending tube is set to be equal to the ratio between d1 and d2 which are intervals between the bending slits, it is possible to cause the proximal end side of the bending portion 7 to bend in a maximum bending state, at up to a curvature identical to a curvature on the distal end side of the bending portion 7.

Thus, the endoscope 1 can easily adapt the shape of the bending portion 7 to the complicated shape of the body cavity inner wall such as the bronchus through a small amount of operation of the bending lever 13 provided in the operation section 3, and it is easier to adjust the bending of the bending portion 7 during a bending operation. For this reason, the endoscope 1 can improve operability even more when bending the bending portion 7.

### (Modification)

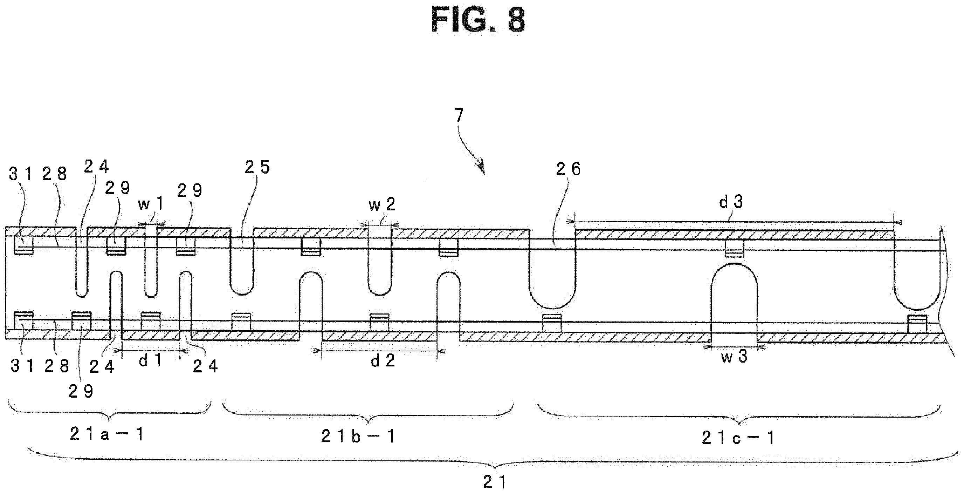

Next, modifications of the present embodiment will be described using the accompanying drawings. Fig. 8 to Fig. 12 illustrate pattern differences in an arrangement or shape of bending slits provided in the bending tube 21.

As for regularity in a pattern, as is understood from the following modifications, an interval between neighboring slits and a width of each of a plurality of slits among the plurality of slits provided in the longitudinal axis direction of a super-elastic tube vary along the longitudinal axis direction of the super-elastic tube.

### (Modification 1)

First, modification 1 will be described using Fig. 8.

An example has been described in the above-described embodiment where two regions (the first and second bending tube regions 21a and 21b) are provided on the distal end side and the proximal end side of the bending tube 21 and bending slits having different widths in the longitudinal axis direction of the bending tube are provided at different intervals for the respective regions of the bending tube 21.

In contrast, the present modification will describe a configuration in which a region to be provided for the bending tube 21 is divided into three stages.

That is, as shown in Fig. 8, a first bending tube region 21a is set on the distal end side of the bending tube 21 of the present modification, and the first bending tube region 21a is provided with the plurality of bending slits 24 whose width in the longitudinal axis direction of the bending tube is set to w1 which are arranged side by side at an interval of d1. Furthermore, a second bending tube region 21b is set closer to the proximal end side than the first bending tube region 21a of the bending tube 21 and the second bending tube region 21b is provided with the plurality of bending slits 25 whose width in the longitudinal axis direction of the bending tube is set to w2 which is wider than the width of the bending slits 24 which are arranged side by side at an interval of d2. Furthermore, a third bending tube region 21c is set on the proximal end side and the third bending tube region 21c is provided with bending slits 26 whose width in the longitudinal axis direction of the bending tube is set to be wider than the width of the bending slits 25 which are arranged side by side at an interval of d3.

The widths (w1, w2 and w3) of the bending slits (24, 25 and 26) in the longitudinal axis direction of the bending tube are set to be wider on the proximal end side than on the distal end side and the intervals (d1, d2 and d3) between the neighboring respective bending slits are set to be wider on the proximal end side than on the distal end side. Furthermore, the ratio among widths w1, w2 and w3 of the respective bending slits (24, 25, and 26) is set to be equal to the ratio among intervals d1, d2 and d3 of the bending slits.

That is, between the neighboring bending slits, the ratios of interval to width are set to be d1:w1=d2:w2=d3:w3.

Note that the widths of the bending slits and the intervals between the bending slits may also be designed so as to vary in multiple stages according to an application or the like.

By changing the widths of the bending slits and the intervals between the bending slits in a plurality of stages, it is possible to bend the distal end side more effectively and preferentially in accordance with the application or purposes or the like and also improve operability at the same time.

Components, actions and effects other than those described above are similar to those of the aforementioned embodiment.

### (Modification 2)

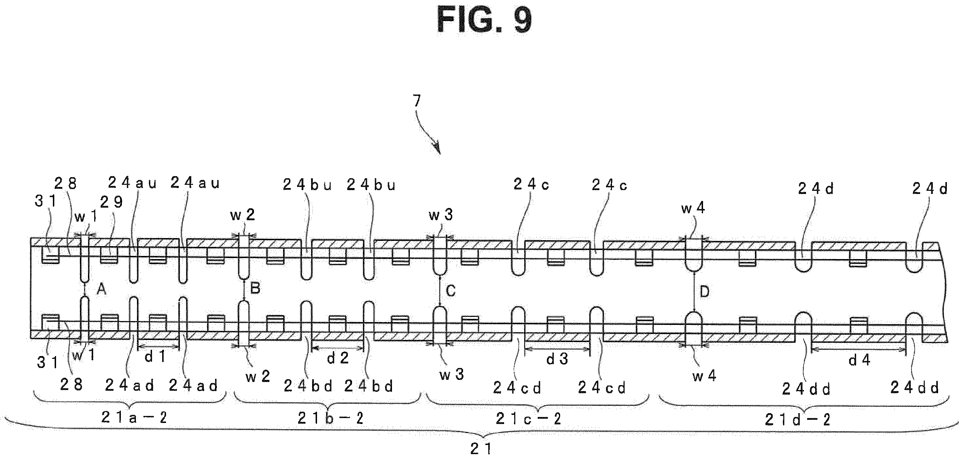

Next, a second modification will be described using Fig. 9.

Here, as shown in Fig. 9, the present modification will describe a configuration in which the bending tube 21 is provided with a first bending tube region 21a-2, a second bending tube region 21b-2, a third bending tube region 21c-2, and a fourth bending tube region 21d-2 from the distal end side.

The first bending tube region 21a-2 is provided with a plurality of bending slits 24au and bending slits 24ad on the upper side and on the lower side of the bending tube 21 respectively. The second bending tube region 21b-2 is provided with a plurality of bending slits 24bu and bending slits 24bd on the upper side and on the lower side of the bending tube 21 respectively. The third bending tube region 21c-2 is provided with a plurality of bending slits 24cu and bending slits 24cd on the upper side and on the lower side of the bending tube 21 respectively. The fourth bending tube region 21d-2 is provided with a plurality of bending slits 24du and bending slits 24dd on the upper side and on the lower side of the bending tube 21 respectively.

In the present modification, the bending slits 24au, 24bu, 24cu and 24du on the upper side in the bending direction and the bending slits 24ad, 24bd, 24cd and 24dd on the lower side in the bending direction are provided at positions opposite to each other.

Furthermore, lengths of the respective bending slits 24au to 24dd extending in the circumferential direction of the bending tube 21 are set so as to differ from one bending tube region to another. More specifically, regarding the length of each bending slit 24, the lengths of the bending slits 24au and 24ad provided in the first bending tube region 21a are longest, and the length is set to decrease in order of the second, third and fourth bending tube regions 21b, 21c and 21d.

Thus, the respective intervals between the bending slits 24au, the bending slits 24bu, the bending slits 24cu and the bending slits 24du provided on the upper side of the bending tube 21 and the bending slits 24ad, the bending slits 24bd, the bending slits 24cd and the bending slits 24dd provided on the lower side of the bending tube 21 are set so as to sequentially increase from the distal end side toward the proximal end side as indicated by A, B, C and D in Fig. 9.

That is, the lengths of the slits in the diameter direction on the distal end side are longer than on the proximal end side.

A slit width w1 set for each of the bending slits 24au and 24ad of the first bending tube region 21a-2, a slit width w2 set for each of the bending slits 24bu and 24bd of the second bending tube region 21b-2, a slit width w3 set for each of the bending slits 24cu and 24cd of the third bending tube region 21c-2 and a slit width w4 set for each of the bending slits 24du and 24dd of the fourth bending tube region 21d-2 are set to increase in the direction from the bending tube region on the distal end side toward the bending tube region on the proximal end side.

Furthermore, an interval d1 between the neighboring bending slits 24au and 24ad on the same slit row in the first bending tube region 21a-2, an interval d2 between the neighboring bending slits 24bu and 24bd on the same slit row in the second bending tube region 21b-2, an interval d3 between the neighboring bending slits 24cu and 24cd on the same slit row in the third bending tube region 21c-2 and an interval d4 between the neighboring bending slits 24du and 24dd on the same slit row in the fourth bending tube region 21d-2 are set to increase in the direction from the bending tube region on the distal end side toward the bending tube region on the proximal end side.

The ratios of width to interval are designed to be w1:d1=w2:d2=w3:d3=w4:d4 as in the case of the aforementioned embodiment.

Components, actions and effects other than those described above are substantially similar to those of the aforementioned embodiment. In this case, according to the present modification, the lengths of the bending slits 24au and 24ad on the distal end side are set to be longest and the length is set to decrease in order of the bending slits 24bu and 24bd, the bending slits 24cu and 24cd, the bending slits 24du and 24dd, and it is thereby possible to more effectively decrease the bending rigidity of the bending tube 21 on the distal end side compared to the proximal end side and more effectively start bending the bending portion 7 from the distal end side first.

### (Third Modification)

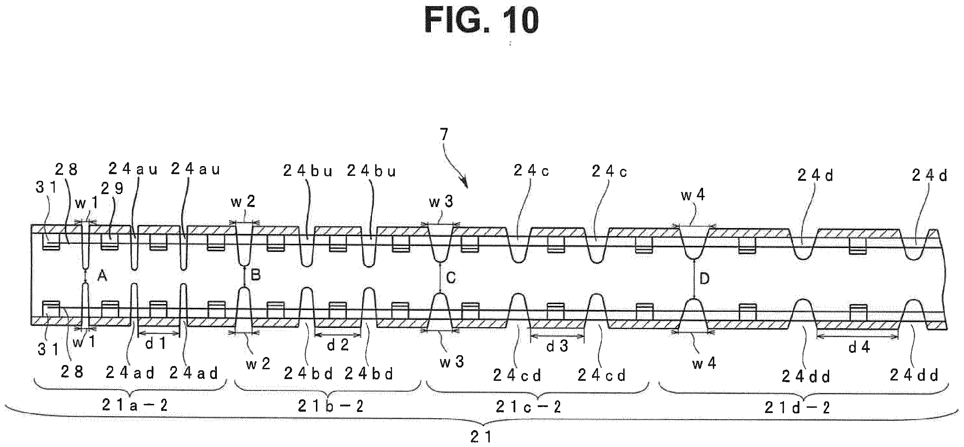

Next, a third modification will be described using Fig. 10.

As shown in Fig. 10, the present modification changes shapes of bending slits compared to the above-described second modification. That is, the bending slits 24au and the bending slits 24ad, the bending slits bu and the bending slits bd, the bending slits cu and the bending slits cd, and the bending slits du and the bending slits dd according to the modification are formed into roundish-rhombic shapes in a plan view, with the widths in the longitudinal axis direction expanding from both ends toward the center.

Note that the slit widths w1, w2, w3 and w4 shown in Fig. 10 indicate the widths in the central part where the bending slits are widest.

Components, actions and effects other than those described above are similar to those of the second modification.

### (Fourth Modification)

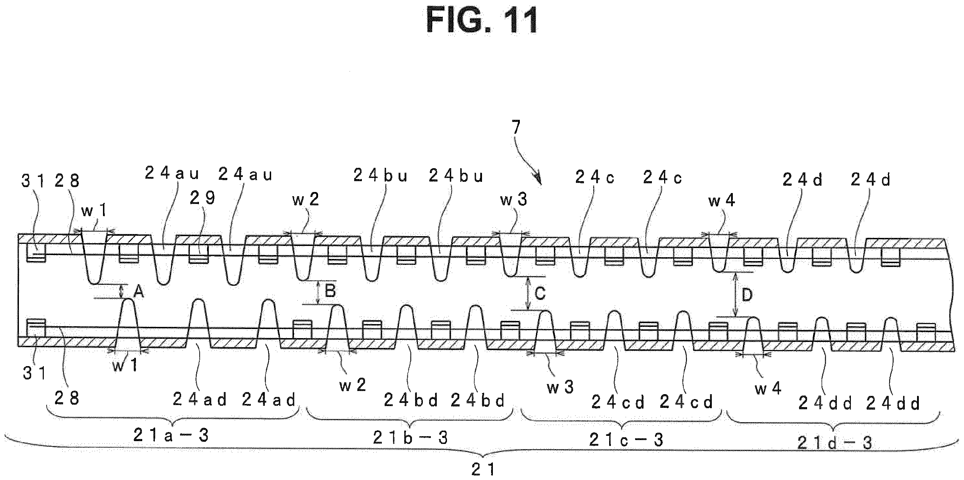

A fourth modification will be described using Fig. 11.

As shown in Fig. 11, shapes of bending slits are assumed to be similar to those of modification 3 and a plurality of bending slits located up and down are alternately arranged at upper and lower positions in a direction orthogonal to the longitudinal direction of the bending tube 21.

Note that in the present modification, first to fourth bending tube regions 21a-3 to 21d-3 correspond to the above-described first to fourth bending tube regions 21a-2 to 21d-2.

Components, actions and effects other than those described above are similar to those of the other embodiments including the present embodiment.

### (Fifth Modification)

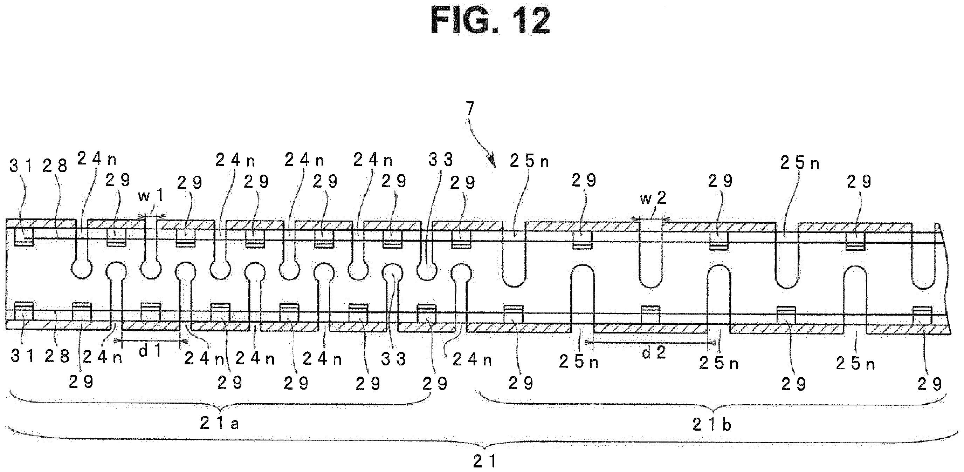

Next, a fifth modification will be described using Fig. 12.

A bending slit 24n shown in Fig. 12 represents the bending slit shown in Fig. 3 changed in shape.

The shape of the bending slit 24n in Fig. 12 is based on the shape of the bending slit 24 shown in Fig. 3 in which slits are formed a shape including into a circular shape having a greater opening area than other parts on an end portion side in the diameter direction and the circle-shaped portion constitutes a stress dispersion portion 33.

With the provision of such stress dispersion portions 33 and the adoption of bending slits in such a shape, it is possible to disperse stress on the slits when performing a bending motion and prevent the occurrence of cracking in the bending tube due to stress concentration on the slits.

The configuration other than the shape of the bending slits 24n is similar to that of the present embodiment.

Note that the present invention is not limited to the above-described each embodiment, but various modifications and alterations can be made, and such modifications and alterations also fall within the technological scope of the present invention. It goes without saying that components of the above-described each embodiment may be combined as appropriate.

The present application claims priority based on Japanese Patent Application No. 2014-203508, filed in Japan on October 1, 2014, the disclosure of which is incorporated in the specification, claims and drawings of the present application by reference.

## Claims

1. An endoscope bending tube having a longitudinal axis that extends from a distal end side to a proximal end side,
the endoscope bending tube comprising a plurality of bending slits configured to penetrate from an outer circumferential surface side to an inner circumferential surface side and extend in a circumferential direction,
wherein the plurality of bending slits are opened in rows at positions on one side and another side independently in a direction orthogonal to the longitudinal axis direction, and two or more bending tube regions along the longitudinal axis direction are formed of slit rows made up of the plurality of bending slits opened at the positions on the one side and the other side, and
a width of each of the bending slits of the slit rows of the bending region located on the distal end side is set to be smaller than a width of each of the bending slits of the slit rows of the bending region located on the proximal end side, and an interval between neighboring bending slits of the slit rows of the bending region located on the distal end side is set to be smaller than an interval between neighboring bending slits of the slit rows of the bending region located on the proximal end side.

2. The endoscope bending tube according to claim 1, wherein the endoscope bending tube is formed of a super-elastic alloy.

3. The endoscope bending tube according to claim 1, wherein a ratio between the width of each of the bending slits and the interval between the neighboring bending slits on the slit rows in a bending region located on the distal end side is substantially equal to a ratio between the width of each of the bending slits and the interval between the neighboring bending slits on the slit rows in a bending region located on the proximal end side.

4. The endoscope bending tube according to claim 1, wherein the plurality of bending slits formed to be opened at positions on the one side and the other side are formed at positions opposite to each other or in the alternate positions.

5. The endoscope bending tube according to claim 1, wherein a length in a diameter direction of the bending slits in a bending region located on the distal end side is set to be longer than a length in a diameter direction of the bending slits in a bending region located on the proximal end side.

6. The endoscope bending tube according to claim 1, wherein the bending slits are formed so as to expand toward a center of the slits in a tapered shape and become narrower toward an end.

7. The endoscope bending tube according to claim 1, wherein a stress dispersion portion is formed at an end portion of the bending slits in a diameter direction.

8. An endoscope comprising the endoscope bending tube according to any one of claims 1 to 7.
